Europäisches Patentamt

European Patent Office

Office européen des brevets

(11) Publication number: **0 393 990 A2**

# (12) EUROPEAN PATENT APPLICATION

(21) Application number: **90304110.1**

(22) Date of filing: **17.04.90**

(51) Int. Cl.⁵: **G01N 33/543, G01N 33/53**

(30) Priority: **19.04.89 US 340246**

(43) Date of publication of application:
**24.10.90 Bulletin 90/43**

(84) Designated Contracting States:
**DE FR IT**

(71) Applicant: **PALL CORPORATION**
**30 Sea Cliff Avenue**
**Glen Cove New York 11542(US)**

(72) Inventor: **Matkovich, Vlado I.**
**P.O Box 783**
**Glen Cove, New York 11542(US)**

(74) Representative: **Knott, Stephen Gilbert et al**
**MATHISEN, MACARA & CO. The Coach**
**House 6-8 Swakeleys Road**
**Ickenham Uxbridge Middlesex UB10 8BZ(GB)**

(54) **Multilayered diagnostic device and method.**

(57) An immunodiagnostic test device for use in detecting analytes in a liquid sample comprising a liquid impervious housing with an opening through which the liquid sample may be applied, a continuous, liquophilic, multilayer member such as a membrane disposed within the housing, the member having a flap or portion to which the liquid sample is applied and the other layers providing for efficient absorption and drawing of liquid down through the membrane flap.

FIG. IB

## MULTILAYERED DIAGNOSTIC DEVICE AND METHOD

The present invention is directed to a diagnostic device and method of use. More particularly, this invention is directed to an immunoassay diagnostic device including a microporous material configured in a continuous multilayer arrangement to assure optimal efficiency and function in drawing a liquid sample through the porous structure.

Various diagnostic test devices are presently employed to detect an analyte in a liquid sample. Among the tests performed in such devices are ELISA assays, RNA and DNA hybridization assays, and microbiological assays. These diagnostic test devices often include a receptacle (or a retaining means), acting as a reservoir for the various reagents required, and a microporous membrane situated in the receptacle. The microporous membrane generally functions in one of two ways: (1) it may act directly as the reactive layer for detecting the presence or absence of specific analyte or (2) it may function to catch and trap particles on its surface with the detection reaction taking place on the trapped particles. The membrane surface on which the reaction takes place, or where the particles are trapped, is considered its upper or "top" surface. Normally, juxtaposed directly below the lower surface of the membrane is a separate construct of a porous absorbent material which pulls the liquids down through the membrane via capillary action as the membrane becomes saturated.

Repeatable and even flow through the membrane is required if test results are to be consistent. Further, rapid flow through the membrane is required if the test is to be of practical application. If irregular flow patterns develop in the membrane or if excessive flow time is experienced, false positives or false negatives can occur. To achieve optimal flow characteristics, effective absorption is a requirement. However, there exists a critical problem which can impede effective adsorption. This critical problem can arise from the trapping of air or gas bubbles between the membrane layer and the absorbent material, often in an irregular fashion.

Trapping of air or gas bubbles can arise due to the basic construction of such a diagnostic device. In its application, the liquid sample must first wet the membrane retained in the device and then flow through the depth of the membrane to communicate with the absorbent pad below. As the absorbent pad becomes wet, capillary forces increase and provide the motive force to pull subsequent liquid through the membrane. The weak link in this chain is the communication between the membrane and the absorbent pad. Due to slight differences from one sample to another or variations in the intimacy of contact between the membrane and the absorbent pad, the liquid front emerging from the membrane may not reach the absorbent pad in a uniform manner. As a result, air bubbles may be trapped between the membrane and the pad during this initial wetting. Once trapped, these air bubbles cannot escape, and, for the remainder of the test, effectively block liquid flow in the areas of the membrane which lie above them. Since current absorbent pad diagnostic devices typically rely on the vertical component of capillary action to draw the liquid sample through the membrane layer, any air trapped between the membrane and the absorbent pad can seriously impede, if not completely block, the flow of liquid through the membrane layer. These blocked areas will not behave like the areas which permit flow, and may result as noted above in false positives, false negatives, or simply indeterminate readings.

In designs such as those described above, steps are often taken to enhance the communication between the membrane and the absorbent pad. Interlayers are added whose fluffy fibrous structures attempt to fill and bridge any gaps between the membrane and the absorbent pad. However, intimacy between all layers is still a requisite for proper function and may be compromised by slight differences in assembly from one device to the next. Inadequate compression between the layers can also result in poor communication between layers (even with the additional fluffy layers interposed), whereas excessive compression can bulge the membrane layer away from the lower structures, also resulting in similar degraded interlayer communication.

The present invention provides for an immunoassay diagnostic test device for use in detecting analytes in a liquid sample comprising: a liquid impervious housing with an opening through which the liquid sample may be applied; a member disposed within the liquid impervious housing, which member is a continuous, microporous structure configured in a multilayer arrangement, the member having a flap to which the liquid sample may be applied. The present invention also provides for a method for detecting an analyte in a liquid sample comprising applying the liquid sample to the flap of the member in the test device described above.

The diagnostic device may further comprise means for venting displaced air from the liquid impervious receptacle and the member may comprise a liquophilic material. Suitable liquophilic materials may consist of the nylon membranes, glass fiber mats, low protein absorbing nylon membranes, non-fluorescing membranes, and hydrophilic webs of synthetic polymeric fibers. Furthermore, the flap of the member may be disposed directly below and across the opening in the housing, thereby forming the base of a liquid receptacle in the test device. The member may also include means for binding a biospecific molecule, and may further include an optical barrier layer positioned between the flap and the layer of the member adjacent the flap.

In the subject invention, the use of a continuous membrane structure provides at least one additional pathway of liquid flow communication which is uninterruptable as it is parallel to and along the membrane surfaces. In addition, the use of a continuous membrane offers economic advantages in the manufacture and assembly of a large number of diagnostic devices, since such a process is more readily automated when employing a single construct as opposed to multiple discrete components which must later be assembled.

The present invention is directed to a diagnostic test device for detecting analytes in liquid samples and to a method of using such a diagnostic device. More particularly, a diagnostic test device is provided employing a continuous microporous structure configured in a multilayer arrangement to circumvent the problems discussed above. The continuous microporous structure optimizes the function of the device as it serves both as the reaction or capture medium and as the liquid absorbent means.

A diagnostic test device embodying the invention can comprise a liquid impervious housing or retaining means which possesses an opening through which a liquid sample is applied. Within the housing, a continuous membrane configured in a multilayer arrangement is disposed, preferably with a vent for displaced air as liquid enters the device.

An immunodiagnostic test device embodying the invention can comprise a liquid impervious housing which houses a pleated liquophilic membrane. The layer of membrane which receives the reagent (or "sample") and faces the aperture or opening in the housing is hereinafter referred to as the "primary membrane flap" or "flap". The primary membrane flap may be treated in such a way that biospecific molecules will bind to its surface or, as one alternative, left untreated and used as a trap for particles to which biomolecules are preattached. Beneath and continuous with the primary membrane flap are pleats or layers of the liquophilic membrane, serving as the absorbent material which draws liquid down through the primary membrane flap via capillary action.

Unlike conventional absorbent pad diagnostic test devices whose operation depends on a minimum of two components (but more typically three components), the device described above employs a one-piece continuous membrane. Furthermore, liquid absorption is not simply reliant on a front of liquid moving through the absorbent from the top to bottom through the depth of the membrane. Rather, liquid communication and subsequent absorption will simultaneously occur along the length of the membrane, i.e., along its folds or pleats.

Since the liquid flow in the subject invention will occur along the membrane itself, i.e., the liquid will move along the membrane from the primary membrane flap and thence from one layer to the next in a direction generally parallel and along the plane of each layer, there is less likelihood of a trapped air bubble completely blocking the liquid pathway as can be observed in the absorbent pad devices of the prior art where the membrane and absorbent material are of two separate constructs. Due to the continuous nature of the membrane, any gas bubbles introduced into the system can be bypassed and thus will not impede the efficiency of the absorption process and, concomitantly, the accuracy of the assay.

Figure 1A is a perspective view of a first reagent receiving primary membrane flap, with the subsequent continuous folds of membrane positioned below (shown in expanded form) to act as the absorbent.

Figure 1B is a side view in cross section of the membrane of Figure 1A in a completed assembly.

Figure 1C is a perspective view of a first reagent receiving primary membrane flap, and the subsequent continuous folds of membrane positioned below (shown in expanded form) to act as the absorbent with an optical barrier positioned between the primary membrane flap and the adjacent layer of the pleated membrane.

Figure 2 is a perspective view of a second embodiment of a reagent receiving primary membrane flap, and the continuous layers of membrane configured in a spiral wrap positioned below the flap to act as the absorbent.

Figure 3 is a side view of a third embodiment of a reagent receiving primary membrane portion, and two sequences comprising the subsequent continuous folds of membrane positioned below to act as the absorbent.

Figure 4 is a perspective view of a fourth embodiment of a reagent receiving primary membrane flap, with the subsequent continuous layers of membrane in a spiral wrap configuration positioned below the flap to act as the absorbent.

Figure 5 is a side view of a fifth embodiment of a reagent receiving primary membrane flap, with the subsequent continuous folds of membrane positioned below to act as the absorbent.

The wettability or liquophilicity of a solid structure, e.g., a membrane, is a function of that structure's critical surface energy and the surface tension of the applied liquid. If the critical surface energy is at least as high as the surface tension of the liquid, the liquid will spontaneously wet the solid structure, which may be termed "liquophilic" with respect to that liquid. For example, a porous membrane having a critical surface energy of 72 dynes/cm or higher will be wetted by water which has a surface tension of 72 dynes/cm and, therefore, tend to freely pass aqueous solutions, i.e., the membrane is "hydrophilic". The capability of a porous structure (e.g., a membrane) to be wetted by a liquid can be determined by placing a drop of the liquid on the porous structure. The angle of contact provides a quantitative measure of wetting. A very high angle of contact indicates poor wetting and may be used to characterize a "liquophobic" material, while a zero angle of contact defines complete or perfect (liquophilic) wetting. Materials used in the subject invention as the wettable or liquophilic porous absorbent structure are characterized by being readily or spontaneously wetted by the applied liquid and have a low angle of contact with the applied liquid. Indeed, when a drop of a test liquid(s) is placed on the wettable or liquophilic layer of the porous structure, the drop of liquid penetrates the layer and provides a low angle of contact with the upper surface of the porous structure.

Suitable materials for use as the wettable layer include those materials which are generally inert toward the solvents, reagents, and other substances typically present either in analyte-containing samples or test reagent solutions used in particular diagnostic tests. Examples of such materials for use in the present invention are wettable forms of polyolefins, polyamides, polyesters, and copolymers of monomers of the aforementioned, and include polyethylene, polypropylene and copolymers of ethylene or propylene, and other olefins. Such other olefins include $\alpha,\beta$-ethylenically unsaturated alkenes. Exemplary of such other olefins is 1-octene. More particularly, materials may include nylon membranes, glass fiber mats, low protein absorbing nylon membranes, non-fluorescing membranes, and hydrophilic webs of synthetic polymeric fibers. Preferred are the nylon membranes described in U.S. Patent No. 4,340,479 which may be neutral or have a positive or negative charge.

The thermoplastic materials used in preparing the liquophilic fibers used in some of the materials of the present invention are in most instances liquophobic and typically hydrophobic. To render the fibers suitable for the present invention, a wetting or surface active agent is combined with the thermoplastic polymer used to form the fibers. The wettable fibers or filaments are prepared by compounding a thermoplastic polymer with at least one, and preferably only one, surface active agent. The term "surface active agent" is intended to include wetting agents and surfactants generally. However, the term is not meant to suggest that the surface active agent is formed only as a coating on the surface of the fibers. Rather, because of the way in which the thermoplastic polymer and surfactant are blended and the fibers are formed, some of the surfactant is believed to be contained within the fibers. The resulting fibers and the non-woven porous structures formed therefrom are liquophilic (and typically hydrophilic), liquid absorbent, and have a low affinity for proteinaceous materials.

As used herein, "analyte" refers to any substance or substances being analyzed for or determined by a diagnostic test. The presence of an analyte is typically indicated by the appropriate detection and/or measurement of a chemical or physical property after reaction or complex formation between the analyte and a detection reagent. Properties which may be observed include color changes, radioactivity or enzyme reactions. The terms "reagent", "test reagent", "detection reagent", and like terms refer both to substances reacting directly with an analyte as well as to substances used to convert either another substance or the analyte to a substance suitable for providing an indication, typically an optical indication, of the presence of the analyte. The term includes simple chemical substances, including elements and compounds, of an ionic or molecular nature as well as more complex or macromolecular structures, such as proteinaceous materials, and includes substances of a biological nature such as antigens, antibodies, enzymes, antibody-enzyme conjugates, haptens, receptors, lectins, gene probes, and the like, as well as viruses and bacteria. The analyte may also be one or more of the substances falling within the definition of a reagent. In the case of antibodies, reagents and analytes may each be monoclonal or polyclonal antibodies. Additionally, the analyte may include drugs, peptides, cells, and organelles. A reagent may additionally include buffers, indicator molecules, and substrates.

As used herein, "multilayer" or "multilayer arrangement" refers to a continuous member constructed and arranged by folding, pleating, spiral wrapping, or the like such that there are multiple layers of the member in contact with adjacent layers of the member. It does not refer to a structure of multiple layers simply bonded together to form a multiple layer composite although such a structure can be used as a starting material in preparing the multilayer arrangement of this invention.

As indicated above, the microporous structures employed in the present invention are liquid absorbent and quite wettable. The latter property is reflected by the Critical Wetting Surface Tension (CWST), which is the surface tension of a liquid that is just capable of wetting a porous structure. The microporous structures of the present invention can be prepared to provide suitable wettability for any liquid employed. However, since aqueous solutions are used most frequently in diagnostic tests, the porous structures generally exhibit CWST values suitably of at least 70 to 72 dynes/cm and some, particularly the non-woven, porous, hydrophilic, liquid absorbent webs described above, have values above 100 dynes/cm.

The pore rating selected for the material employed as the multilayer structure will depend on the particular application to which the diagnostic test device will be put. The porous structure has preferred pore dimentions which would generally be characterized as microporous. The nature of the liquid sample being tested will, in many instances, determine the pore rating of the microporous structure to be used. However, pore ratings are suitably between 0.04 and 10 microns, preferably 0.2 to 5 microns and most preferably 0.45 to 3 microns.

Diagnostic test devices embodying the present invention can comprise a continuous, microporous structure configured in a multilayer arrangement. As a class, pleated structures are preferred in part because of their relative ease of manufacture. Other multilayer structures, such as spirally wound ones, are also contemplated.

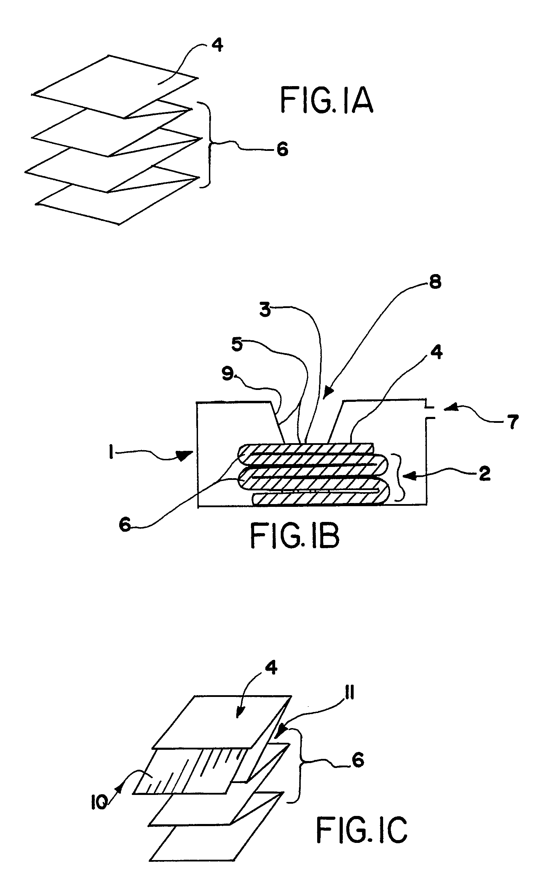

As illustrated in Figure 1A, the simplest form of a pleated structure comprises an upper primary membrane flap 4, followed sequentially by a number of absorbent layers or folds 6 created by folding the continuous microporous structure back and forth upon itself. It should be noted that while the terms "membrane" and "membrane flap" are used hereinafter, a variety of microporous structures including fibrous webs or mats may be used in the continuous multilayer structures of embodiments of this invention.

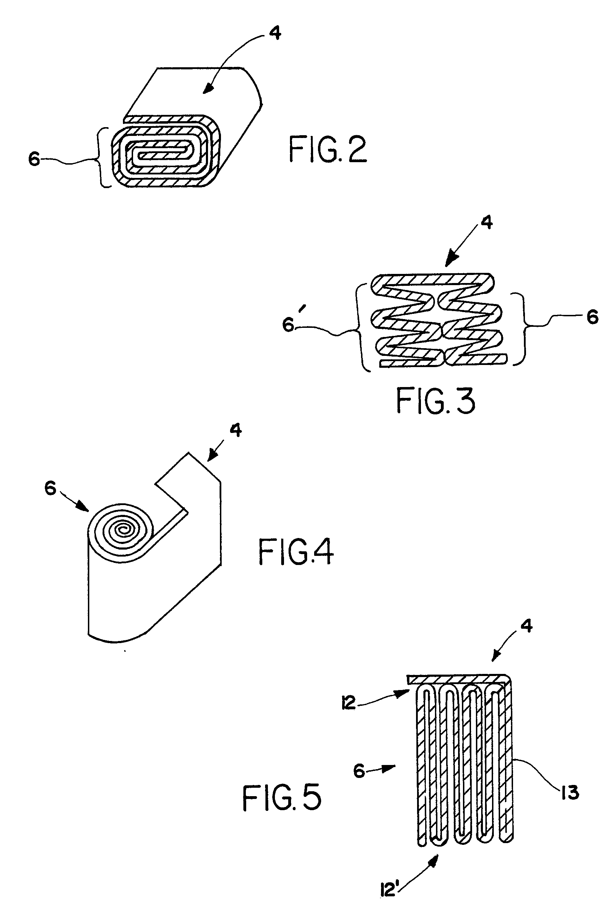

A diagnostic test device embodying the present invention and utilizing a pleated structure is illustrated in Figure 1B. As shown in Figure 1B, a liquid impervious housing is provided with a pleated membrane structure serving as the primary membrane flap and the absorbent material. For convenience, the term "flap" or "member flap" is used here and throughout the description of the preferred embodiments section to refer to the layer of the continuous member which receives the reagent albeit with certain of the configurations the portion of the continuous member which receives the reagent is not a flap in the conventional sense of one end being free, e.g., see Figure 3. Also, the embodiments discussed hereinafter should be considered in the context of the need in each instance for a housing or liquid impervious receptacle associated with the described multilayered structure.

As shown in Figure 1B, a liquid impervious housing 1 surrounds the liquophilic membrane structure 2. The housing 1 is provided with an upper opening 8 from which walls 9 depend downwardly toward the inside of the housing 1. As determined by the shape of the opening 8, the walls 9 may be a single wall of a generally circular or elliptical cross-section or a series of connected walls which form a rectangular, pentagonal, etc. cross-section. The wall(s) 9 define at their base an aperture or opening 3 through which a liquid sample may be introduced to the test device. The primary membrane flap 4 is the top layer of the liquophilic membrane structure 2 and is disposed across the aperture or opening 3 such that the wall(s) 9 and the membrane flap 4 define a liquid receptacle, indicated generally by 5. The liquophilic membrane structure 2 is continuous and consists of a series of folds or pleats 6 which create the subsequent absorbent layers. The absorbent layers possess upper and lower surfaces which are in intimate contact with adjacent layers and allow the absorbent layers to serially draw the liquid sample through the bottom surface of the primary membrane flap. An air vent 7, which may be a simple hole, allows air (or gas) to escape as the air is displaced from the region surrounding the membrane structure by the liquid entering the device. If desired, the air vent 7 may be covered with a porous liquophobic material to prevent liquid escape from the interior of the housing.

The liquid sample may be applied to the upper surface of the primary membrane flap 1 of the pleated membrane, i.e., placed in the liquid receptacle 5. The subsequent layers, being in intimate contact with the lower surface of the primary membrane flap, draw the liquid sample down through the depth of the membrane in an essentially vertical direction as this initial layer becomes saturated. Simultaneously, a component of liquid flow in the primary membrane flap is developing in the transverse direction, parallel and along the length of the membrane surface. This flow component proceeds into the layer below as a result of the continuous liquid pathway offered by the structure. Thus, in the event of a gas bubble impediment or "air locking" (where an air or gas bubble blocks liquid flow), the liquid sample can bypass the bubble utilizing this transverse component of flow, and the liquid is drawn along the length of the liquophilic membrane structure along the folds or pleats. Once the liquid component which is flowing transversely has bypassed the air lock, this flow component can recombine with the essentially vertical flow component. Because of the multilayered configuration of the structure, these twin components of flow, vertical and transverse, can occur at all levels of the pleated membrane structure, the effects of "air locking" at any level can be circumvented.

When the primary membrane surface is used as a direct binding site for biomolecules which are specific for the analyte to be detected, the preferred continuous membrane is a microporous, hydrophilic, nylon 66 membrane material having high binding capacity, uniformity, controlled pore size, and high surface area available from Pall Corporation as **Biodyne**TM and described in U.S. Patent No. 4,340,479.

When the continuous membrane is to be used as a direct binding site for biomolecules and a covalent chemical bond is desired or is necessary, the preferred membrane is a derivatized nylon 66 membrane, available from Pall Corporation as a chemically active membrane known as **Immunodyne**TM described in U.S. Patent No. 4,693,985 or alternatively as **Immunodyne**TM **II** described in U.S. Patent No. 4,886,836.

For capture of particulates on the primary membrane flap, e.g., for the capture of whole or of parts of bacteria, or for the capture of latex or polystyrene beads on which biospecific molecules have previously been immobilized, a membrane which is inherently resistant to nonspecific binding is preferred. Such a membrane is available from Pall Corporation as **LoProdyne**TM and described in EPO Patent Publication No. 0 272 841 and in U.S. Patent No. 4,886,836.

For applications where the analyte will be detected by fluorescence, a non-fluorescing membrane is preferred, e.g., such as the one available from Pall Corporation as **Biodyne**TM **NF** as described in U.S. Patent No. 4,837,162.

Another preferred embodiment of the present invention includes a mat or web rendered hydrophilic. The mat or web described comprises a liquid absorbent, non-woven material formed from thermoplastic, liquophilic fibers having a low non-specific affinity for amide group-containing substances. Examples of such materials for use as these mats or webs include wettable forms of polyolefins, polyamides, polyesters, and copolymers of monomers of the aforementioned, and include polyethylene, polypropylene and copolymers of ethylene or propylene, and other olefins. The thermoplastic materials used in preparing the liquophilic fibers used in some of the materials of the present invention are in most instances liquophobic and typically hydrophobic. To render the fibers suitable for the present invention, a wetting or surface active agent is combined with the thermoplastic polymer used to form the fibers.

The wettable fibers or filaments are prepared by compounding a thermoplastic polymer with at least one, and preferably only one, surface active agent. The pore ratings of these mats or webs are typically in the range of 0.5 to 20 microns.

Also preferred for applications including capture of whole bacteria, of parts of bacteria, or of latex or polystyrene spheres on which specific biospecific molecules have been previously immobilized, is a glass fiber mat or web made from a mixture of glass fibers and resins and subsequently fabricated by conventional paper-making techniques and available from Pall Corporation.

In certain applications of the subject invention, the reagents which are passed through the primary membrane flap, to react with and ultimately give a color indication, thus demonstrating the presence of a specific analyte in the initial liquid sample, may themselves commingle below the primary membrane flap, react, and produce a color. The color produced by this commingling is specious and not an indication of the presence of the analyte under detection. Several of the membranes mentioned earlier characteristically become somewhat translucent upon wetting however. Thus, in applications where a specious color may develop in the layers below the primary membrane flap, some form of optical isolation may be necessary.

Preferred for this optical barrier is the hydrophilic web described above. The barrier may also be formed of a separate sheet of a glass fiber mat or web as described above. In either case, the optical barrier or barrier layer 10 is preferably interposed between the bottom of the primary membrane flap 4 and the adjacent layer 11 of the pleated continuous membrane structure as illustrated in Figure 1C.

In other applications of the subject invention, the sample being applied may contain particulates, gels, or cellular material, and consequently may tend to block the upper surface of the primary membrane flap to flow. With conventional absorbent pad devices, such material needs to be prefiltered prior to application. For this class of samples, the subject invention can be employed in a variation which eliminates this need for prefiltration in certain cases.

In these applications, the device may be supplied with the primary membrane flap's lower surface initially not in intimate contact with the remainder of pleats or folds below. The sample is then applied to the underside of the primary membrane flap, allowed to adsorb, and then the primary membrane flap is forced into juxtaposition with the adjacent pleats or folds below and retained in that position for the remainder of the test. In this manner, the primary membrane flap functions as its own prefilter, and reaction between the prefiltered sample and the biospecific molecules preattached to the membrane can take place. As subsequent liquids and reagents are added to the reservoir above the membrane flap to complete the reaction, liquid flow will be impeded by the unwanted particulate or cellular components trapped between the primary membrane flap and the layers below, analogous to air locking. However, flow will be unaffected in the transverse direction due to the maintained uninterrupted communication between the primary membrane flap and the adjacent layers below as described in detail above. Thus the reaction can be completed and the color change on the upper surface of the primary membrane flap noted.

As depicted in Figure 2, a liquid impervious housing (as depicted in Figure 1B) may be used to surround the liquophilic membrane structure with an aperture provided through which a liquid sample may be introduced into the test device. The embodiment depicted in Figure 2 may be used in the manner described above where the sample being applied may contain particulates, gels, or cellular material which may block the upper surface of the primary membrane flap 4. That is, the sample may be applied to the underside of the primary membrane flap 4, allowed to absorb, and the flap then forced into contact with the adjacent absorbent pleats or folds 6 for the remainder of the test.

In the embodiment depicted in Figure 2, a primary membrane flap or layer 4 may be fashioned from a portion of the end of the liquophilic membrane structure and disposed across the aperture to form the base of the liquid receptacle as shown in Figure 1B. The liquophilic membrane structure itself is continuous and consists of a spirally wrapped roll of liquophilic material which as shown in Figure 2 is somewhat flattened with the central axis of the spiral structure running parallel to the plane of the primary membrane layer or flap 4. The absorbent layers 6 which are subsequently formed below possess outer and inner surfaces (with respect to the central axis of the spiral structure) which are in intimate contact with adjacent layers and allow the absorbent layers 6 to serially draw the liquid sample from the primary membrane flap in the direction of the base of the receptacle.

The sample may be administered to the upper or outer surface of the primary membrane flap or layer 4 of the rolled membrane, and the subsequent layers, being in intimate contact with the lower surface of the primary membrane flap or layer 4, draw the liquid sample down through the depth of the membrane in an essentially vertical direction as this initial layer becomes saturated. Simultaneously, a component of flow in the primary membrane layer 4 is developing in the transverse direction along the length of the membrane surface, due to the structure's continuous communication with the spiral layers below. Thus, in the event of a gas bubble impediment or "air locking", the liquid sample can bypass the bubble utilizing this transverse component of flow, as the liquid is drawn along the length of the rolled liquophilic membrane. In most cases, once the liquid flowing transversely has bypassed the air lock, this flow component will recombine with the essentially vertical flow component. These components of flow may occur at all levels of the rolled membrane structure, circumventing the effects of air-locking.

As depicted in Figure 3, the primary membrane flap 4 may be formed at an intermediate portion in the membrane structure. This layer can then be disposed across the lower part of the aperture, forming the base of the liquid receptacle as depicted in Figure 1B. The liquophilic membrane structure itself is continuous and consists of two series of folds or pleats which create two series of pleated layers 6 and 6′, i.e., the subsequent two absorbent layer sequences 6 and 6′. Each absorbent layer sequence comprises absorbent layers with widths only a portion of the width of the primary membrane layer or flap 4; however, when the measure of each width is combined with the measure of the corresponding layer of the other absorbent layer sequence, the widths of these combined absorbent layers 6 will preferably be substantially equal to the width of the primary membrane flap 4, albeit their combined widths may be either greater or less than the width of the primary membrane layer 4. Each absorbent layer 6 and 6′ possesses upper and lower surfaces which are in intimate contact with adjacent layers and allow the absorbent layers 6 and 6′ to serially draw the liquid sample through the bottom surface of the primary membrane flap 4.

The sample may be administered to the upper surface of the primary membrane layer 4 of the pleated membrane, and the subsequent absorbent layers 6 and 6′, being in intimate contact with the lower surface of the primary membrane flap 4, draw the liquid sample down through the depth of ·the membrane in an essentially vertical direction as this initial layer becomes saturated. Simultaneously, two components of flow in the primary membrane layer 4 are developing in the transverse direction, parallel and along either length of the membrane surface, due to the primary membrane layer's continuous communication with both series of layers below. Thus, in the event of a gas bubble impediment or "air locking", the liquid sample can bypass the bubble utilizing a transverse component of flow, as the liquid is drawn along the length of the liquophilic membrane structure following or along the folds.

In most cases, once the liquid flowing transversely has bypassed the air lock, this flow component will recombine with the essentially vertical flow component. These twin components of flow, vertical and transverse, may occur at all levels of the pleated membrane structure; thus, the effects of "air locking" at any level can be circumvented.

As depicted in Figure 4, the primary membrane flap 4 may be the portion formed from the end of the liquophilic membrane structure and can be disposed across the lower part of the aperture forming the base of the liquid receptacle as depicted in Figure 1B. One end of the liquophilic membrane structure is wider than the remainder of the membrane's length. The end with this additional width of material may be folded over to form the primary membrane layer 4, continuous with the remainder of the membrane. The liquophilic membrane structure itself is continuous and consists of a spirally wrapped roll of liquophilic material with the central axis of the cylinder running perpendicular to the plane of the primary membrane flap. The absorbent layers 6 below the flap possess inner and outer surfaces (with respect to the cylinder's central axis) which are in intimate contact with adjacent layers and allow serial absorption of the liquid sample from the adjacent absorbent layers towards the central axis. The absorbent layers 6 run perpendicular to the primary membrane flap 4, and the upper edges of these absorbent layers are in intimate contact with the lower surface of the primary membrane layer and allow the absorbent layers to draw the liquid sample through the bottom surface of the primary membrane layer 4 and into the absorbent layers 6 towards the base of the housing.

The sample may be administered to the upper surface of the primary membrane layer 4 of the rolled membrane, and the upper edges of the absorbent layers 6, being in intimate contact with the lower surface of the primary membrane layer 4, draw the liquid sample down through the depth of the membrane in an essentially vertical direction as this initial layer becomes saturated. Simultaneously, a component of flow in the primary membrane flap is developing everywhere tangent to the surface of the membrane surface, due to the primary membrane layer's continuous communication with the absorbent layers 6. The flow along the length of the primary membrane flap 4, as it saturates the outer absorbent layer formed along its length, may (1) continue along the length of the membrane or (2) pursue a pathway through the depth of each absorbent layer 6 towards the central axis of the cylinder.

The primary membrane layer 4 in the configuration depicted in Figure 4 can act as its own prefilter just as described above with regard to the embodiment of Figure 2.

In the embodiment of the present invention depicted in Figure 5, the primary membrane flap 4 is the top layer of the liquophilic membrane structure which can be disposed across the lower part of the aperture, forming the base of the liquid receptacle as depicted in Figure 1B. The liquophilic membrane structure itself is continuous and has a series of folds or pleats which create the subsequent absorbent layers 6. These absorbent layers 6 possess upper and lower folds which create crests 12 and 12', respectively, with the absorbent layers 6 running perpendicular to the surface of the primary membrane layer 4. The upper peaks 12 of the absorbent layers 6 are in intimate contact with the primary membrane flap 4, and allow the absorbent layers 6 to draw the liquid sample through the bottom surface of the primary membrane layer 4 along the planes of the absorbent layers 6.

The sample may be administered to the upper surface of the primary membrane layer 4 of the pleated membrane, and the upper crests 12 of the absorbent layers 6 below, being in intimate contact with the lower surface of the primary membrane layer 4, draw the liquid sample down through the depth of the membrane in an essentially vertical direction as this initial layer becomes saturated. Simultaneously, a component of flow in the primary membrane flap 4 can develop along the length of the membrane surface, due to the structure's continuous communication with the absorbent layers 6. The flow along the length of the primary membrane layer 4, as it saturates the first absorbent layer 13 formed along its length, may (1) continue along the length of the membrane or (2) pursue a transverse pathway through the depth of each absorbent layer 6. This transverse flow component may then recombine with the essentially vertical flow component. These twin components of flow, vertical and transverse, may occur at all levels of the pleated membrane structure; thus, the effects of "air locking" can be reduced.

The liquophilic membrane structure described in each of the embodiments may initially comprise a single continuous layer of membrane material or may include a folded, multilayered arrangement prior to being configured into the absorbent, allowing for additional flow paths in each of the embodiments described above.

Many different embodiments of this invention may be made without departing from the scope and spirit thereof; and, therefore, the invention is not intended to be limited except as indicated in the appended claims.

## Claims

1. An immunoassay diagnostic test device for use in detecting analytes in a liquid sample comprising:

a liquid impervious housing with an opening through which the liquid sample may be applied;

a member disposed within the liquid impervious housing, which member is a continuous, microporous structure configured in a multilayer arrangement, the member having a flap to which the liquid sample may be applied.

2. The diagnostic test device of claim 1 further comprising means for venting displaced air from the liquid impervious receptacle.

3. The diagnostic test device of claim 1 wherein the member comprises a liquophilic material.

4. The diagnostic test device of claim 3 wherein the liquophilic material is selected from the group consisting of nylon membranes, glass fiber mats, low protein absorbing nylon membranes, non-fluorescing membranes, and hydrophilic webs of synthetic polymeric fibers.

5. The diagnostic test device of claim 1 wherein the flap of the member is disposed directly below and across the opening in the housing, thereby forming the base of a liquid receptacle in the test device.

6. The diagnostic test device of claim 1 wherein said member includes means for binding a biospecific molecule.

7. The diagnostic test device of claim 1 wherein an optical barrier layer is positioned between the flap and the layer of the member adjacent the flap.

8. The diagnostic test device of claim 1 wherein the member is in pleated form with an optical barrier layer positioned between the flap and the layer of the member adjacent the flap.

9. The diagnostic test device of claim 1 wherein the flap is so constructed and arranged that a liquid sample may be applied to the side which will be in contact with the next adjacent layer.

10. The diagnostic test device of claim 1 wherein the member is in a pleated configuration.

11. A method for detecting an analyte in a liquid sample comprising applying the liquid sample to the flap of the member in the test device of claim 1.

12. A method for detecting an analyte in a liquid sample comprising applying the liquid sample to the under side of the flap of the member in the diagnostic test device of claim 9, allowing the sample to adsorb and contacting the under side of the flap with the next adjacent layer of the member.

FIG.IA

FIG.IB

FIG.IC

EP 0 393 990 A2

FIG.2

FIG.3

FIG.4

FIG.5